(19) 

Europäisches
Patentamt

European
Patent Office

Office européen
des brevets

(11) **EP 3 960 205 A1**

(12) **EUROPEAN PATENT APPLICATION**

(43) Date of publication:
   **02.03.2022 Bulletin 2022/09**

(21) Application number: **21185996.2**

(22) Date of filing: **16.07.2021**

(51) International Patent Classification (IPC):
   **A61L 2/00** (2006.01)    **A61L 9/20** (2006.01)
   **A61L 2/10** (2006.01)

(52) Cooperative Patent Classification (CPC):
   **A61L 9/20; A61L 2/10;** A61L 2202/11;
   A61L 2202/14; A61L 2202/25; A61L 2209/11

(84) Designated Contracting States:
   **AL AT BE BG CH CY CZ DE DK EE ES FI FR GB
   GR HR HU IE IS IT LI LT LU LV MC MK MT NL NO
   PL PT RO RS SE SI SK SM TR**
   Designated Extension States:
   **BA ME**
   Designated Validation States:
   **KH MA MD TN**

(30) Priority: **25.08.2020 JP 2020141843**

(71) Applicant: **Ushio Denki Kabushiki Kaisha
   Tokyo-to
   100-8150 (JP)**

(72) Inventor: **OKUMURA, Yoshihiko
   Tokyo, 100-8150 (JP)**

(74) Representative: **Tomerius, Isabel
   Lang & Tomerius
   Patentanwaltspartnerschaft mbB
   Rosa-Bavarese-Strasse 5
   80639 München (DE)**

(54) **INACTIVATION APPARATUS AND INACTIVATION METHOD**

(57)     To provide an inactivation apparatus and an inactivation method that are capable of inactivating microorganisms and viruses more efficiently using ultraviolet (UV) light having a wavelength of 200 nm to 230 nm, disclosed herein is an inactivation apparatus for inactivating microorganisms and/or viruses on a surface of an object, comprising: an ultraviolet light irradiation unit having a light emission surface configured to emit light including ultraviolet light having a wavelength that inactivates the microorganisms and/or viruses; and a controller unit configured to control irradiation of the light by the ultraviolet light irradiation unit, the ultraviolet light included in the light emitted from the light emission surface being ultraviolet light having a center wavelength of 200 nm to 230 nm, and the controller unit controlling the ultraviolet light irradiation unit to irradiate the surface of the object with the ultraviolet light to form a region, on the surface of the object, in which irradiance of the ultraviolet light having the wavelength of 200 nm to 230 nm becomes equal to or greater than 10 $\mu$W/cm$^2$.

FIG. 6

EP 3 960 205 A1

**Description**

CROSS-REFERENCE TO RELATED APPLICATIONS

This application is related to and claims priority of Japanese patent application No. 2020-141843, filed on August 25, 2020, of which the disclosure including the specification, drawings and abstract is incorporated herein by reference in its entirety.

TECHNICAL FIELD

The present invention relates to an inactivation apparatus and method for inactivating harmful microorganisms and viruses.

BACKGROUND ART

Conventionally, in order to prevent the spread of infectious diseases caused by harmful microorganisms (bacteria, molds, or the like) and viruses, microorganisms and viruses floating in the space, as well as microorganisms and viruses attached to various places such as floors, walls, and surfaces of objects, have been inactivated by irradiating them with ultraviolet (UV) light.

For example, Patent Literature 1 (Laid-open Publication of Japanese Patent Application No.2018-130131 A) discloses an indoor sterilization apparatus that is mounted above the room and capable of irradiating UV light horizontally, obliquely downward and downwardly in the room.

In addition, as means of inactivating microorganisms and viruses using UV light, it is common to use UV light having a wavelength of 254 nm, which is so-called germicidal or sterilizing rays. DNA (deoxyribonucleic acid) possessed by microorganisms and viruses has an absorption band of UV light at around the wavelength of 260 nm. It has been known that irradiating DNA with the UV light having a wavelength of 254 nm causes photochemical reactions such as hydration phenomena, dimer formation, and decomposition, resulting in the inactivation of microorganisms (e.g., bacteria and molds) and viruses.

However, care must be taken not to irradiate humans and animals with the light of 254 nm, which serves as a germicidal ray, because it is harmful to humans and animals.

LISTING OF REFERENCES

PATENT LITERATURE

PATENT LITERATURE 1: Laid-open Publication of Japanese Patent Application No. 2018-130131 A

PATENT LITERATURE 2: Japanese Translation of PCT International Application Publication No. 2018-517488 A

SUMMARY OF THE INVENTION

PROBLEMS TO BE SOLVED BY THE INVENTION

In recent years, the global spread of infectious diseases has drawn attention to inactivation apparatuses that use the UV light for inactivating microorganisms and viruses.

It has also been reported that UV light having a wavelength of 200 nm to 230 nm can selectively inactivate microorganisms and viruses without harming human or animal cells. Such inactivation apparatuses using the UV light having a wavelength of 200 nm to 230 nm are strongly expected to be a new type of inactivation apparatus that is harmless to humans and animals. For example, Patent Literature 2 (Japanese Translation of PCT International Application Publication No. 2018-517488 A) discloses the selective inactivation of viruses without harming human cells by UV irradiation having a wavelength of 200 nm to 230 nm.

The present invention has been made in order to solve the above-mentioned problems and an object thereof is to provide an inactivation apparatus and an inactivation method that are capable of inactivating microorganisms and viruses more efficiently using ultraviolet (UV) light having a wavelength of 200 nm to 230 nm.

SOLUTION TO PROBLEMS

In order to solve the above mentioned problems, according to one aspect of the present invention, there is provided an inactivation apparatus for inactivating microorganisms and/or viruses on a surface of an object, comprising: an ultraviolet light irradiation unit having a light emission surface configured to emit light including ultraviolet light having a wavelength that inactivates the microorganisms and/or viruses; and a controller unit configured to control irradiation of the light by the ultraviolet light irradiation unit, the ultraviolet light included in the light emitted from the light emission surface being ultraviolet light having a center wavelength of 200 nm to 230 nm, and the controller unit controlling the ultraviolet light irradiation unit to irradiate the surface of the object with the ultraviolet light to form a region, on the surface of the object, in which irradiance of the ultraviolet light having the wavelength of 200 nm to 230 nm becomes equal to or greater than 10 $\mu$W/cm$^2$.

According to another aspect of the present invention, there is provided an inactivation apparatus for inactivating microorganisms and/or viruses floating in a target space, comprising: an ultraviolet light irradiation unit having a light emission surface configured to emit light including ultraviolet light having a wavelength that inactivates the microorganisms and/or viruses; and a controller unit configured to control irradiation of the light by the ultraviolet light irradiation unit, the ultraviolet light included in the light emitted from the light emission surface being ultraviolet light having a center wavelength of 200 nm to 230 nm, and the controller unit controlling the ultraviolet light irradiation unit to irradiate the target space with the ultraviolet light to form a region in which irradiance of the ultraviolet light having the wavelength of 200 nm to 230 nm becomes equal to or greater than 10 $\mu$W/cm$^2$ at a distance of 20 cm from the light emission surface.

The present inventors have found that the inactivation effect of microorganisms and viruses in the inactivation treatment using ultraviolet light having a wavelength of 200 nm to 230 nm, which are not harmful to human and animal cells, greatly depends on the irradiance (in other words, light intensity per unit time) as well as the integrated light intensity (in other words, cumulative light amount). Furthermore, the present inventors have also found that the inactivation effect in terms of the irradiance gradually transitions with a boundary of 10 $\mu$W/cm$^2$.

As described above, when the target of the inactivation treatment is a surface of an object, a region where the ultraviolet irradiance of 200 nm to 230 nm wavelength is equal to or greater than 10 $\mu$W /cm$^2$ is formed on the surface of the object. Likewise, when the target of the inactivation treatment is a space, a region where the ultraviolet irradiance of 200 nm to 230 nm wavelength is equal to or greater than 10 $\mu$W/cm$^2$ is formed at a distance of 20 cm from the light emission surface. As a result, it makes it possible to more effectively inactivate microorganisms and viruses per the same integrated light intensity.

In the above inactivation apparatus, the ultraviolet irradiation unit may emit ultraviolet light having a center wavelength of 222 nm.

In this case, even in a situation where humans or animals are present, it makes it possible to appropriately suppress the adverse effects on the human bodies and animals due to the ultraviolet irradiation.

Furthermore, in the above inactivation apparatus, the controller unit may control lighting by the ultraviolet light irradiation unit such that an irradiance value of the ultraviolet light by the ultraviolet light irradiation unit varies over time or periodically between a first irradiance value and a second irradiance value that is lower than the first irradiance value.

In this case, the lighting may be controlled such that, at the lighting time when the irradiance value is higher (i.e., first irradiance value), a region where the ultraviolet irradiance is equal to or greater than 10 $\mu$W/cm$^2$ is formed on the surface of the object or in the target space, while at the lighting time when the irradiance value is lower (i.e., second irradiance value), the ultraviolet irradiance is controlled to be less than 10 $\mu$W/cm$^2$ for the region. In this way, by alternately repeating high irradiance value and low irradiance value, it makes it possible to lengthen the period until the integrated light intensity reaches a predetermined amount as compared to the case where continuous lighting is performed with high ultraviolet irradiance. Thus, it makes it possible to extend the period of time during which the inactivated environment can be maintained so as not to exceed the maximum allowable daily ultraviolet exposure amount to the human body, which is specified according to the wavelength of the ultraviolet light to be irradiated. In particular, when forming a region where the ultraviolet irradiance is equal to or greater than 10 $\mu$W/cm$^2$ on the surface of the object or in the target space, the integrated light intensity in the region or its surrounding regions will reach the predetermined amount in a shorter period of time. For this reason, by providing a period of lower irradiance, it makes it possible to perform inactivation with higher ultraviolet irradiance while suppressing the integrated light intensity of ultraviolet light irradiated in a given period.

Yet furthermore, in the above inactivation apparatus, the controller unit may control the ultraviolet light irradiation unit to alternately repeat an emitting operation and a non-emitting operation of the light by the ultraviolet light irradiation unit and to intermittently perform light emission from the light emission surface.

In this way, when light emitting operation and non-emitting operation are alternately repeated, so-called intermittent lighting, it makes it possible to lengthen the period until the integrated light intensity reaches a predetermined amount as compared to the case of continuous lighting with the same ultraviolet irradiance. In other words, the inactivated environment (or the period during which inactivation takes place) is maintained by irradiating with ultraviolet light intermittently. Thus, it makes it possible to extend the period of time during which the inactivated environment can be maintained so as not to exceed the maximum allowable daily ultraviolet exposure amount to the human bodies, which is specified according to the wavelength of the ultraviolet light to be irradiated. In particular, when forming a region where the ultraviolet irradiance is equal to or greater than 10 $\mu$W/cm$^2$ on the surface of the object or in the target space, the integrated light intensity in the region or its surrounding regions will reach the predetermined amount in a shorter period of time. For this reason, by controlling the alternating repetition of light emitting operation and non-emitting operation, it makes it possible to perform inactivation with high ultraviolet irradiance while suppressing the integrated light intensity of ultraviolet light irradiated in a given period. In addition, by controlling the alternating repetition of light emitting operation and non-emitting operation, the usable life of the light source (i.e., the time until the light source needs to be replaced) can be extended as compared to the case of continuous lighting.

Furthermore, the ultraviolet light irradiation unit emits ultraviolet light having a center wavelength of 200 nm to 230 nm, which can effectively inhibit photoreactivation of bacteria. Therefore, even if the light of 300 nm to 500 nm wavelength is irradiated during the non-light-emitting operation period after the light emitting operation, the bacteria can be prevented from being photo-reactivated during the non-light-emitting operation period so as to maintain the inactivation effect of the light emitting operation. In other words, it makes it possible to attain the equivalent inactivation effect to that of continuous lighting.

According to yet another aspect of the present invention, there is provided an inactivation method of inactivating microorganisms and/or viruses on a surface of an object, comprising the steps of: controlling an ultraviolet light irradiation unit to emit light having a center wavelength of 200 nm to 230 nm, as ultraviolet light having a wavelength that inactivates the microorganisms and/or viruses, from a light emission surface of the ultraviolet light irradiation unit; and irradiating the surface of the object with the ultraviolet light to form a region, on the surface of the object, in which irradiance of the ultraviolet light having the wavelength of 200 nm to 230 nm becomes equal to or greater than 10 $\mu$W/cm$^2$.

According to yet another aspect of the present invention, there is provided an inactivation method of inactivating microorganisms and/or viruses floating in a target space, comprising the steps of: controlling an ultraviolet light irradiation unit to emit light having a center wavelength of 200 nm to 230 nm, as ultraviolet light having a wavelength that inactivates the microorganisms and/or viruses, from a light emission surface of the ultraviolet light irradiation unit; and irradiating the target space with the ultraviolet light to form a region in which irradiance of the ultraviolet light having the wavelength of 200 nm to 230 nm becomes equal to or greater than 10 $\mu$W/cm$^2$ at a distance of 20 cm from the light emission surface.

In this way, when the target of the inactivation treatment is a surface of an object, a region where the ultraviolet irradiance of 200 nm to 230 nm wavelength is equal to or greater than 10 $\mu$W /cm$^2$ is formed on the surface of the object. Likewise, when the target of the inactivation treatment is a space, a region where the ultraviolet irradiance of 200 nm to 230 nm wavelength is equal to or greater than 10 $\mu$W/cm$^2$ is formed at a distance of 20 cm from the light emission surface. As a result, it makes it possible to more effectively inactivate microorganisms and viruses per the same integrated light intensity.

Furthermore, in the above inactivation method, lighting by the ultraviolet light irradiation unit may be controlled such that an irradiance value of the ultraviolet light by the ultraviolet light irradiation unit varies over time or periodically between a first irradiance value and a second irradiance value that is lower than the first irradiance value.

In this case, the lighting may be controlled such that, at the lighting time when the irradiance value is higher (i.e., first irradiance value), a region where the ultraviolet irradiance is equal to or greater than 10 $\mu$W/cm$^2$ is formed on the surface of the object or in the target space, while at the lighting time when the irradiance value is lower (i.e., second irradiance value), the ultraviolet irradiance is controlled to be less than 10 $\mu$W/cm$^2$ for the region. In this way, by alternately repeating high irradiance value and low irradiance value, it makes it possible to lengthen the period until the integrated light intensity reaches a predetermined amount as compared to the case where continuous lighting is performed with high ultraviolet irradiance. Thus, it makes it possible to extend the period of time during which the inactivated environment can be maintained so as not to exceed the maximum permissible daily ultraviolet exposure amount to the human bodies, which is specified according to the wavelength of the ultraviolet light to be irradiated. In particular, when forming a region where the ultraviolet irradiance is equal to or greater than 10 $\mu$W/cm$^2$ on the surface of the object or in the target space, the integrated light intensity in the region or its surrounding regions will reach the predetermined amount in a shorter period of time. For this reason, by providing a period of lower irradiance, it makes it possible to perform inactivation with high ultraviolet irradiance while suppressing the integrated light intensity of ultraviolet light irradiated in a given period.

Yet furthermore, in the above inactivation method, the ultraviolet light irradiation unit may be controlled to alternately repeat an emitting operation and a non-emitting operation of the light by the ultraviolet light irradiation unit and to intermittently perform light emission from the light emission surface.

In this way, when light emitting operation and non-emitting operation are alternately repeated, so-called intermittent lighting, it makes it possible to lengthen the period until the integrated light intensity reaches a predetermined amount as compared to the case of continuous lighting with the same ultraviolet irradiance. In other words, the inactivated environment (or the period during which inactivation takes place) is maintained by irradiating with ultraviolet light intermittently. Thus, it makes it possible to extend the period of time during which the inactivated environment can be maintained so as not to exceed the maximum allowable daily ultraviolet exposure amount to the human bodies, which is specified according to the wavelength of the ultraviolet light to be irradiated. In particular, when forming a region where the ultraviolet irradiance is equal to or greater than 10 $\mu$W/cm$^2$ on the surface of the object or in the target space, the integrated light intensity in the region or its surrounding regions will reach the predetermined amount in a shorter period of time. For this reason, by controlling the alternating repetition of light emitting operation and non-emitting operation, it makes it possible to perform inactivation with high ultraviolet irradiance while suppressing the integrated light intensity of ultraviolet light irradiated in a given period. In addition, by controlling the alternating repetition of light emitting operation and non-emitting operation, the usable life of the light source (i.e., the time until the light source needs to be replaced) can be extended as compared to the case of continuous lighting.

It should be noted that, in the present invention, the term "inactivation" refers to the killing of microorganisms and viruses (or the loss of infectivity or toxicity). The term "irradiance of ultraviolet light having a wavelength of 200 nm to 230 nm" refers to the total irradiance of ultraviolet light in the wavelength band from 200 nm to 230 nm.

## ADVANTAGEOUS EFFECT OF THE INVENTION

According to the present invention, it makes it possible to inactivate microorganisms and viruses more efficiently without harming human or animal cells.

The above mentioned and other not explicitly mentioned objects, aspects and advantages of the present invention will become apparent to those skilled in the art from the following embodiments (detailed description) of the invention by referring to the accompanying drawings and the appended claims.

## BRIEF DESCRIPTION OF DRAWINGS

FIG. 1 is a chart exemplarily illustrating experimental data on the inactivation effect of Staphylococcus aureus.

FIG. 2 is a chart exemplarily illustrating data showing the relationship between irradiance and inactivation effect.

FIG. 3 is a chart exemplarily illustrating data showing the dependence on irradiance for an integrated light intensity of 5 [mJ/cm$^2$].

FIG. 4 is a chart exemplarily illustrating data showing the dependence on irradiance for an integrated light intensity of 10 [mJ/cm$^2$].

FIG. 5 is a schematic diagram illustrating an exemplary configuration of an inactivation apparatus that inactivates objects.

FIG. 6 is a schematic diagram illustrating an exemplary configuration of an inactivation apparatus that inactivates a space.

FIG. 7 is a schematic diagram illustrating an exemplary configuration of an inactivation system incorporating the inactivation apparatus.

FIG. 8 is a time chart illustrating an exemplary intermittent lighting operation of the inactivation apparatus.

FIG. 9 is a chart exemplarily illustrating results of a photoreactivation experiment on bacteria irradiated with ultraviolet light at a wavelength of 254 nm.

FIG. 10 is a chart exemplarily illustrating results of a photoreactivation experiment on bacteria irradiated with ultraviolet light at a wavelength of 222 nm.

FIG. 11 is a chart exemplarily illustrating comparison results between continuous lighting and intermittent lighting at a wavelength of 254 nm.

FIG. 12 is a chart exemplarily illustrating comparison results between continuous lighting and intermittent lighting at a wavelength of 222 nm.

FIG. 13A is a time chart of the Operation Example 1.

FIG. 13B is a time chart of the Operation Example 2.

FIG. 14 is a chart exemplarily illustrating experimental results of the Operation Examples 1 and 2.

FIG.15A is a time chart of the Operation Example 3.

FIG.15B is a time chart of the Operation Example 4.

FIG. 16 is a chart exemplarily illustrating experimental results of the Operation Examples 3 and 4.

FIG. 17A is a time chart of the Operation Example 5.

FIG. 17B is a chart exemplarily illustrating experimental results of the Operation Example 5.

FIG. 18 is a schematic diagram illustrating an exemplary use case of the inactivation apparatus.

FIG. 19 is a schematic diagram illustrating another exemplary use case of the inactivation apparatus.

FIG. 20 is a schematic diagram illustrating yet another exemplary use case of the inactivation apparatus.

FIG. 21 is a schematic diagram illustrating yet another exemplary use case of the inactivation apparatus.

## DESCRIPTION OF EMBODIMENTS

Hereinafter, non-limiting embodiments of the present invention will be described in detail with reference to the accompanying drawings. Among the constituent elements disclosed herein, those having the same function are denoted by the same reference numerals, and a description thereof is omitted. It should be noted that the embodiments disclosed herein are illustrative examples as means for implementing the present invention, and should be appropriately modified or changed depending on a configuration and various conditions of an apparatus to which the present invention is applied, and the present invention is not limited to the following embodiments. Furthermore, it should be noted that all of the combinations of features described in the following embodiments are not necessarily essential to the solution of the present invention.

The present embodiment will describe an inactivation system that inactivates microorganisms and viruses by ultraviolet light (hereinafter referred to as "UV light" or simply referred to as "UV") irradiation in facilities or vehicles where humans or animals are present.

Here, the above facilities can be, for example, offices, commercial facilities, medical facilities, schools, theaters, restaurants, or the like. The above facility may be a closed, semi-closed or unclosed space, such as a hospital room, conference room, restroom, or inside an elevator. The above vehicle may be an automobile, train, bus, airplane, ship, or the like.

The inactivation system according to the present embodiment irradiates a surface or a space in a facility or a vehicle with UV light having a wavelength of 200 nm to 230 nm, which have little adverse effect on the cells of humans and animals, to inactivate microorganisms and viruses that are harmful to humans or animals, at least those that exist on the surfaces and spaces in the facility or vehicle. The "space inside the facility or a vehicle where a human or an animal exists," which is a space where the UV light is irradiated, is not limited to the space where a human or an animal actually exists, but also includes the space where a human or an animal may enter and leave and where no human or animal continuously exist.

Inactivation of microorganisms and viruses using UV light has been verified mainly using UV light having a wavelength of 254 nm, which is a germicidal ray. According to those findings, it has been considered that the inactivation of microorganisms and viruses is expressed in terms of the integrated light intensity (i.e., irradiance * time) of UV light, and the inactivation effect depends on the integrated light intensity (in other words, cumulative light amount).

In other words, it has been considered that doubling the UV light irradiation time would produce the same inactivation effect even if the irradiance (in other words, light intensity per unit time) was reduced to one-half.

However, as a result of intensive investigation by the present inventors, it has been newly found that the inactivation effect of microorganisms and viruses significantly depends on the irradiance as well as the integrated light intensity, at least in the inactivation treatment using UV light having a wavelength of 200 nm to 230 nm, which is not harmful to human and animal cells. This is a new finding that is different from the conventional knowledge.

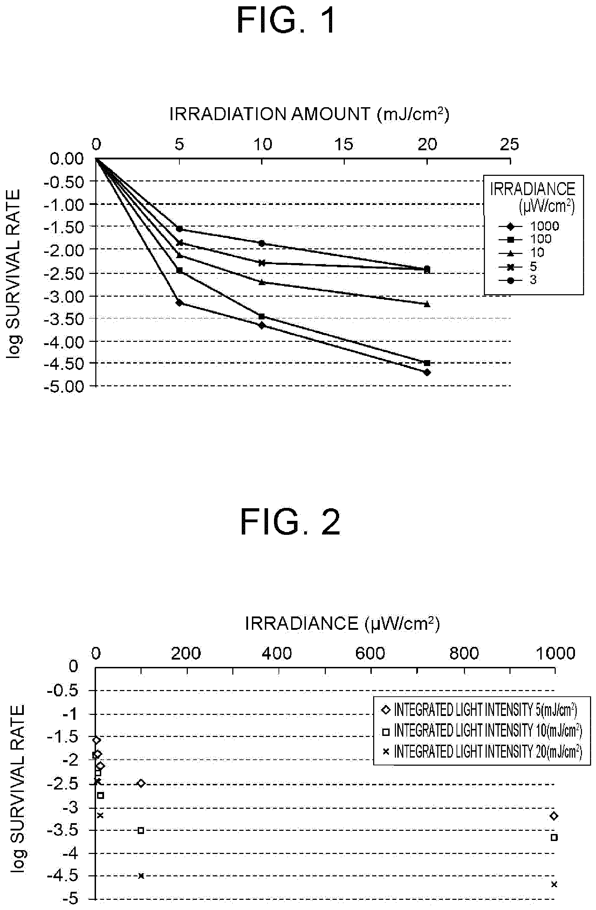

FIG. 1 is a chart exemplarily illustrating experimental data verifying the inactivation effect of Staphylococcus aureus by irradiation with UV light having a center wavelength of 222 nm. Referring to FIG. 1, the horizontal axis denotes the integrated light intensity (i.e., irradiation amount) [mJ/cm$^2$] and the vertical axis denotes the log survival rate of the bacteria. The log survival rate here is expressed by Log (the number of colonies of the bacteria after UV irradiation / the number of colonies of the bacteria before UV irradiation). In FIG.1, the changes in the survival rate of bacteria have been examined under different UV irradiance [$\mu$W/cm$^2$].

The experiment shown in FIG. 1 was carried out according to the following procedure. First, prepare a solution containing 10$^7$ CFU/mL of bacteria, drop 2 to 3 mL of the solution into a petri dish of $\phi$35 mm, and place the petri dish in a predetermined position to be irradiated with UV light. Subsequently, determine the UV irradiance to a predetermined value, adjust the UV irradiation time to achieve a predetermined integrated light intensity, and irradiate the solution in the petri dish with UV light. Subsequently, the UV irradiated solution is seeded onto a standard agar medium, and the medium is incubated at 36 °C (degrees Celsius) for 48 hours, and then the number of colonies present in the medium is counted. By performing the above procedure, the changes in the survival rate of the bacteria were confirmed depending on the irradiance of UV light and the irradiation amount (i.e., integrated light intensity).

As shown in FIG. 1, even if the integrated light intensity is the same, it is understood that the higher the irradiance, the higher the inactivation effect. In other words, it can be observed that the inactivation effect has the dependency on irradiance.

Although FIG. 1 shows the experimental data when the inactivation target is Staphylococcus aureus, it is assumed that the same results can be obtained even when the inactivation target is a virus. Hereinafter, this point will be described below.

Bacteria and viruses share the same mechanism of inactivation in that DNA bindings are damaged by UV irradiation. In addition, when bacteria are inactivated by irradiation with UV light having a wavelength of 200 nm to 230 nm, in particular, by irradiation with UV light having a wavelength of 222 nm, it was found that the repairing damaged DNA (i.e., photoreactivation of bacteria) was not performed even when the bacteria are irradiated with light having a wavelength of 300 nm to 500 nm after UV irradiation. The photoreactivation of bacteria is due to the action of photo-reactivating enzymes (e.g., FAD (flavin adenine dinucleotide)) possessed by bacteria. The UV light having a wavelength of 200 nm to 230 nm, especially the UV light having a wavelength of 222 nm, effectively acts on those photo-reactivating enzymes and has an effect of inhibiting the function of photoreactivation.

On the other hand, viruses are not photo-reactivatable as viruses do not have the above described photo-reactivating enzymes.

For this reason, it can be assumed that the inactivation mechanism of virus is equivalent to the inactivation mechanism of bacteria using UV light having a wavelength of 222 nm, which destroys even photo-reactivating enzymes and inhibits photoreactivation.

As a result, it is presumed that the dependency on irradiance can be confirmed when the inactivation target is a virus, similar to the experimental results shown in FIG. 1.

FIG. 2 is a chart exemplarily illustrating the relationship between irradiance [$\mu$W/cm$^2$] and inactivation effect (i.e., log survival rate of bacteria) for the irradiance range of 0 to 1000 $\mu$W/cm$^2$ for the cases of integrated light intensity of 5 mJ/cm$^2$, 10 mJ/cm$^2$, and 20 mJ/cm$^2$ among the experimental data shown in FIG. 1.

As shown in FIG. 2, the relationship between the irradiance and the inactivation effect is not linear, and it can be observed that there are irradiance regions with strong irradiance dependency and irradiance regions with weak irradiance dependency.

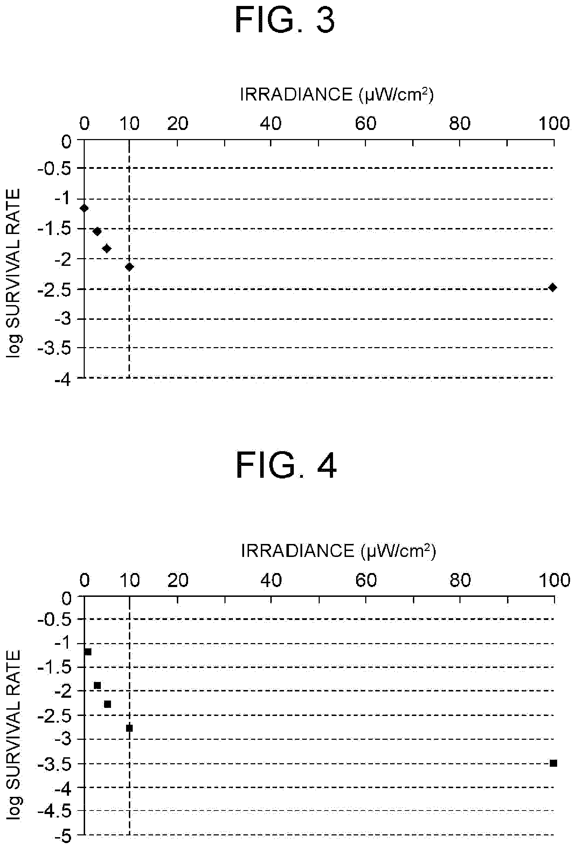

Furthermore, FIG. 3 is a chart exemplarily illustrating the results of examining the relationship between the irradiance [$\mu$W/cm$^2$] and the inactivation effect (i.e., log survival rate of bacteria) when the integrated light intensity is 5 mJ/cm$^2$, for the irradiance range of 0 to 100 $\mu$W/cm$^2$.

As shown in FIG. 3, the inactivation effect gradually decreases in response to changes in the irradiance with 10 $\mu$W/cm$^2$ as a change point (i.e., boundary). In other words, it can be observed that the dependency on irradiance is strong in the irradiance region below 10 $\mu$W/cm$^2$, and the dependency on irradiance is weak in the irradiance region above 10 $\mu$W/cm$^2$.

Here, the change point is a point at which the change in slope exceeds a predetermined threshold.

FIG. 4 is a chart exemplarily illustrating the results of examining the relationship between the irradiance [$\mu$W/cm$^2$] and the inactivation effect (i.e., log survival rate of bacteria) when the integrated light intensity is 10 mJ/cm$^2$ for the irradiance range of 0 to 100 $\mu$W/cm$^2$.

As shown in FIG. 4, it can be seen that when the integrated light intensity is 10 mJ/cm$^2$, a change point lies at an irradiance of 10 $\mu$W/cm$^2$, similarly to the case when the integrated light intensity is 5 mJ/cm$^2$ (as shown in FIG. 3).

It should be noted that, in the above experiment, one type of light source (i.e., lamp) was used, and by changing the distance from the light emission surface of the light source to the bacteria to be inactivated (in this case, Staphylococcus aureus), the irradiance of the UV light irradiated to the bacteria was changed.

However, the same experimental results can be obtained, for example, by changing the irradiance of the UV light irradiated to the bacteria by changing the irradiance of the light emitted from the light source, while the distance from the light emission surface of the light source to the bacteria to be inactivated is kept constant. The same experimental results can be obtained no matter how far the separation distance is at that time.

The inactivation effect is determined by the irradiance of the UV light and the irradiation time. In other words, the degree of inactivation effect is determined by how much UV light is irradiated onto the microorganisms and bacteria. For this reason, what is important in the above experiment is how much irradiance of UV light is irradiated to the microorganisms and bacteria and for how long, and the method to obtain the prescribed irradiance is not limited.

Regardless of how the separation distance from the light source and the irradiance at the light emission surface of the light source are set, the change point in the irradiance dependency is confirmed at an irradiance of 10 $\mu$W/cm$^2$, as shown in FIGs. 3 and 4.

As described above, it has been found that the inactivation effect has the dependency on the irradiance in the inactivation treatment using UV light having a wavelength of 200 nm to 230 nm. Furthermore, it has been also confirmed that the inactivation effect with respect to the irradiance gradually transitions with a boundary of 10 $\mu$W/cm$^2$.

In other words, it has been found that, while, in the irradiance regions lower than 10 $\mu$W/cm$^2$, the variation of the inactivation effect associated with irradiance change is steep (i.e., the dependency on the irradiance is strong), in the irradiance regions at 10 $\mu$W/cm$^2$ or higher, the variation of the inactivation effect associated with irradiance change becomes gentle (i.e., the dependency on irradiance becomes weak).

For this reason, in order to effectively perform the inactivation, it is desirable to perform inactivation at an irradiance equal to or greater than 10 $\mu$W/cm$^2$. It makes it possible to inactivate microorganisms and viruses more effectively even with the same integrated light intensity.

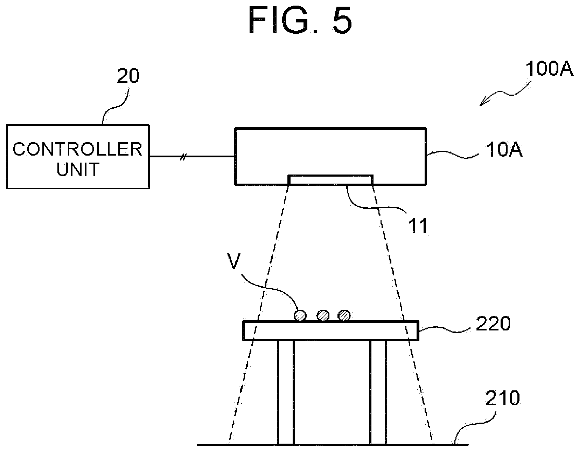

FIG. 5 is a schematic diagram illustrating an exemplary configuration of an inactivation apparatus 100A for inactivating objects.

Referring to FIG. 5, the inactivation apparatus 100A is an inactivation apparatus that inactivates microorganisms and/or viruses on a surface of an object, and includes an ultraviolet light irradiation unit (i.e., UV irradiation unit) 10A and a controller unit 20. The UV light radiation unit 10A has a light emission surface 11 and emits UV light, from the light emission surface 11, having a peak in the wavelength band of 200 nm to 230 nm. As the UV light source, for example, a KrCl excimer lamp that emits UV light having a center wavelength of 222 nm can be used. UV light having a center wavelength of 222 nm is a light that kills bacteria and other organisms but has little adverse effect on human cells.

The controller unit 20 controls the irradiation and non-irradiation of UV light from the UV irradiation unit 10A. More particularly, the controller unit 20 controls the UV irradiation unit 10A such that a region is formed on the surface of the object in which the UV irradiance at a wavelength of 200 nm to 230 nm is equal to or greater than 10 $\mu$W/cm$^2$.

For example, as shown in FIG. 5, when the UV irradiation unit 10A is installed on the ceiling of the facility with the light emission surface 11 as the bottom surface, and the object to be inactivated is a desk 220 installed on the floor 210, a region is formed on the surface of the desk 220 in which the UV irradiance at a wavelength of 200 nm to 230 nm is equal to or greater than 10 $\mu$W/cm$^2$.

This can effectively inactivate bacteria and viruses V attached to the surface of the desk 220.

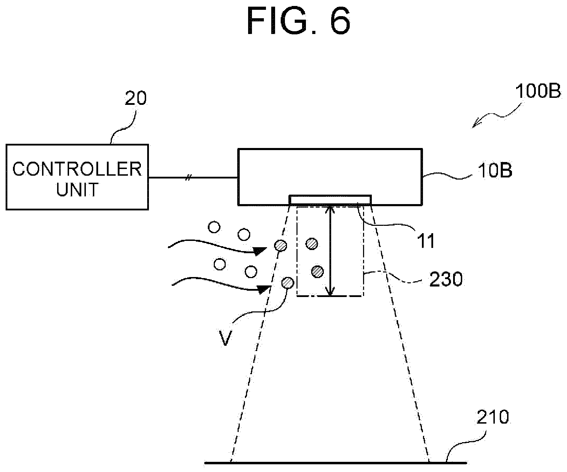

FIG. 6 is a schematic diagram illustrating an exemplary configuration of an inactivation apparatus 100B for inactivating a space.

The inactivation apparatus 100B is an inactivation apparatus that inactivates microorganisms and/or viruses floating in a target space, and includes an ultraviolet light irradiation unit (i.e., UV irradiation unit) 10B and a controller unit 20. The UV irradiation unit 10B is installed on the ceiling of a passageway or room in a facility with the emission surface 11 as the bottom surface to inactivate microorganisms and/or viruses floating above the passageway or room. The UV irradiation unit 10B has a light emission surface 11 and emits UV light having a peak in the wavelength band of 200 nm to 230 nm from the light emission surface 11. As a UV light source, for example, a KrCl excimer lamp that emits UV light having a center wavelength of 222 nm can be used.

The controller unit 20 controls the irradiation and non-irradiation of the UV light from the UV irradiation unit 10B. More particularly, the controller unit 20 controls the UV irradiation unit 10B such that a region is formed in which the irradiance of UV light having a wavelength of 200 nm to 230 nm is equal to or greater than 10 $\mu$W/cm$^2$ at a position with a predetermined distance from the light emission surface 11.

The above separation distance from the light emission surface 11 is a distance in the vertical direction from the light emission surface 11, and the above predetermined distance can be, for example, 20 cm.

The irradiance of the target space can be measured by installing an illuminance meter at a predetermined distance (e.g., 20 cm) in the vertical direction from the light emission surface 11 using a measuring tape, laser, or other dimensional measuring instrument.

For example, as shown in FIG. 6, when the UV irradiation unit 10B is installed on the ceiling of the facility with the light emission surface 11 as the bottom surface and emits UV light downward, a region in which the irradiance of UV light having a wavelength of 200 nm to 230 nm is equal to or greater than 10 $\mu$W/cm$^2$ is formed in the space between the light emission surface 11 and the floor 210 at a position 20 cm vertically downward from the light emission surface 11.

As a result, it makes it possible to ensure that the UV irradiance is equal to or greater than 10 $\mu$W/cm$^2$ in the target space 230 within 20 cm from the light emission surface 11, and to effectively inactivate bacteria and viruses V floating in the target space 230.

It should be noted that the target space 230 is not limited to a space 20 cm from the light emission surface 11. The target space 230 may be a space less than 20 cm from the light emission surface 11, or a space more than 20 cm from the light emission surface 11.

An object (such as the desk 220 shown in FIG. 5) may be disposed in a region irradiated with UV light outside the target space 230 shown in FIG. 6. In this case, even when the surface of the object outside the target space 230 is irradiated with UV light having the irradiance less than 10 $\mu$W/cm$^2$, an incidental inactivation effect can be obtained for microorganisms and viruses attached to the surface of the object.

As described above, the inactivation apparatus 100A, in which the target of the inactivation treatment is an object surface, includes the UV irradiation unit 10A having a light emission surface 11 that emits light including UV light having a wavelength that inactivates microorganisms and/or viruses, and a controller unit 20 that controls the irradiation of light by the UV irradiation unit 10A. Here, the UV light included in the light emitted from the light emission surface 11 is UV light having a center wavelength of 200 nm to 230 nm. The controller unit 20 controls the UV irradiation unit 10A to form a region on the surface of the object where the irradiance of the UV light having a wavelength of 200 nm to 230 nm is equal to or greater than 10 $\mu$W/cm$^2$.

As a result, it makes it possible to effectively inactivate microorganisms and viruses attached to the surface of the object.

Likewise, the inactivation apparatus 100B, in which the target of the inactivation treatment is a space, includes a UV irradiation unit 10B having a light emission surface 11 that emits light including UV light having a wavelength that inactivates microorganisms and/or viruses, and a controller unit 20 that controls the irradiation of light by the UV irradiation unit 10B. Here, the UV light included in the light emitted from the light emission surface 11 is UV light having a center wavelength of 200 nm to 230 nm. The controller unit 20 controls the UV irradiation unit 10B to form a region where the irradiance of the UV light having a wavelength of 200 nm to 230 nm is equal to or greater than 10 $\mu$W/cm$^2$ at a distance of 20 cm from the light emission surface 11.

In this way, the UV irradiance is ensured to be equal to or greater than 10 $\mu$W/cm$^2$ at a distance of 20 cm away from the light emission surface 11. As a result, it makes it possible to effectively inactivate microorganisms and viruses in the targeted space.

When the target of the inactivation treatment is a space, the wider the range where the UV irradiance is equal to or greater than 10 $\mu$W/cm$^2$, the wider the range that can be effectively utilized for space sterilization and inactivation, which is more preferable. For example, it is more preferable that the range where the UV irradiance is equal to or greater than 10 $\mu$W/cm$^2$ is ensured to be 50 cm or higher, 100 cm or higher, 120 cm or higher, or 140 cm or higher.

Furthermore, in the inactivation apparatuses 100A and 100B according to the present embodiment, the controller unit 20 may control the lighting such that the irradiance value of the UV light by the UV irradiation units 10A and 10B varies (fluctuates) over time or periodically between a higher irradiance value (i.e., first irradiance value) and a lower irradiance value (i.e., second irradiance value). In this case, at the lighting time when the irradiance value is high, a region where the UV irradiance is equal to or greater than 10 $\mu$W/cm$^2$ is formed on the object surface or in the target space. On the other hand, at the lighting time when the irradiance value is low, the lighting may be controlled such that the irradiance becomes less than 10 $\mu$W/cm$^2$ for that region. Yet furthermore, the light emitting operation and non-emitting operation by the UV irradiation units 10A and 10B may be alternately repeated, and the light emission from the light emission surface 11 may be intermittently performed, which is so-called intermittent lighting.

According to the ACGIH (American Conference of Governmental Industrial Hygienists) and JIS (Japanese Industrial Standards) Z 8812 (Measurement method of harmful ultraviolet radiation), the allowable limit value (TLV: Threshold Limit Value) for an irradiation amount of UV light to the human body per day (8 hours) is specified for each wavelength. Therefore, when the lighting is kept on at a relatively high irradiance of 10 μW/cm² or more, as in the above described embodiment, there is a possibility that the allowable limit value may be exceeded early.

On the other hand, from the viewpoint of preventing the spread of infection by microorganisms or viruses, it is desirable to maintain the inactivation effect at all times during the period in which humans and animals are travelling.

For this reason, the intermittent lighting, in which UV light irradiation is intermittent, may be performed while setting the irradiance high as described above. In this way, by setting the irradiance high, it makes it possible to attain the higher inactivation effect even with the same integrated light intensity as described above. By performing the intermittent lighting, it makes it possible to lengthen the period until the integrated light intensity reaches the predetermined amount as compared to the case of continuous lighting with the same UV irradiance. In other words, it makes it possible to extend the UV irradiation period as compared to the case of continuous lighting with the same UV irradiance. As a result, it makes it possible to effectively perform inactivation over a longer period of time as compared to the case of continuous lighting. Also, the usable life of the light source (i.e., the time until the light source needs to be replaced) can be extended.

It should be noted that the inactivation apparatus according to the present embodiment is expected to be widely applied to spaces where humans or animals come and go or stay, and to object surfaces which humans or animals may touch. Considering the inactivation of microorganisms and/or viruses, it is effective to create a region on the surface of the object or in the target space where the irradiance of UV having a wavelength of 200 nm to 230 nm is equal to or greater than 10 μW/cm².

However, as described above, according to the ACGIH and JIS Z 8812 (Measurement method of harmful ultraviolet radiation), the allowable limit value (TLV) for the irradiation amount of UV light per day (i.e., 8 hours) to the human body is specified for each wavelength. Thus, when the UV irradiance is too high, the irradiation amount of UV light per day (i.e., 8 hours) reaches the allowable limit value in a relatively short time. Therefore, it becomes difficult to sustainably inactivate spaces where humans or animals come and go or stay. For this reason, it is more desirable that the above described UV irradiance is lower than 2,500 μW/cm² at the highest.

As will be discussed later, according to the current safety standards, the maximum allowable UV exposure amount of 222 nm wavelength for one day (i.e., 8 hours) is set to Dmax = 22 (mJ/cm²). Assuming that the UV irradiance to the surface of the object is increased, and a region with a UV irradiance of 5,000 μW/cm² is formed on the surface of the object, the maximum permissible irradiation time can be ensured for only about 0.5 seconds per hour, on the assumption that humans and animals come and go in the vicinity of the region.

This is also true for the target space. Assuming that the UV irradiance to the target space is increased and a region with a UV irradiance of 5,000 μW/cm² is formed at a distance of 20 cm from the light emission surface of the UV irradiation unit, the maximum permissible irradiation time can be ensured for only about 0.5 seconds per hour, on the assumption that humans and animals come and go in the vicinity of the region. It should be noted, however, this maximum allowable UV exposure amount is no more than the current value and may be changed to a higher exposure in the future. Nevertheless, when the UV irradiance is too high, it will be difficult to ensure the irradiation time.

In view of the above circumstances, it is more desirable to control the UV irradiance to a range not exceeding 2,500 μW/cm², while forming the region where the UV irradiance at a wavelength of 200 nm to 230 nm is equal to or greater than 10 μW/cm² on the surface of the object or in the target space. As far as the UV irradiance is within the range of not exceeding 2,500 μW/cm², an irradiation time of at least one second per hour can be ensured, making it easier to achieve sustainable inactivation.

Hereinafter, an inactivation system for performing intermittent lighting will be described in detail.

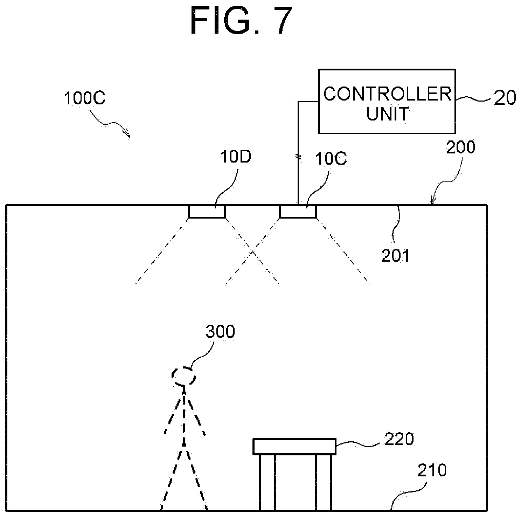

As shown in FIG. 7, the inactivation apparatus 100C, which performs intermittent lighting, includes an ultraviolet irradiation unit (UV irradiation unit) 10C that emits UV light to surfaces and spaces in the facility 200, a light source for illumination 10D, and a controller unit 20.

The UV irradiation unit 10C is installed, for example, on the ceiling 201 in the facility 200. Any UV irradiation unit 10C is deployable as long as it can emit UV light to surfaces and spaces within the facility 200, and the installation position is not particularly limited. For example, the UV irradiation unit 10C may be installed on a wall in the facility 200, or may be supported by an arm stand or the like installed in the facility 200.

The light emitted by the UV irradiation unit 10C includes UV light in the wavelength range of 190 nm to 235 nm, which have less adverse effects on the human bodies.

The UV irradiation unit 10C is equipped with a KrCl excimer lamp that emits UV light having a center wavelength of 222 nm, for example, as a UV light source. The UV irradiation unit 10C may include a wavelength selective filter that transmits only light in the wavelength range of 190 nm to 235 nm and cuts light in other wavelength ranges.

As a wavelength selective filter, for example, an optical filter having dielectric multilayers with HfO₂ and SiO₂ layers can be used. More particularly, the optical filter may have a structure in which a dielectric multilayer film consisting of alternating layers of HfO₂ and SiO₂ is formed on one surface of a substrate made of synthetic quartz (silica) glass, and an AR coating of HfO₂ and SiO₂ layers is applied on the other surface of the substrate.

Alternatively, an optical filter with dielectric mul-

tilayers of $SiO_2$ and $Al_2O_3$ layers can also be used as the wavelength selective filter.

However, when the optical filter with dielectric multilayer film made of $HfO_2$ and $SiO_2$ layers is used as a wavelength selective filter, the total number of layers can be reduced as compared to the case where an optical filter with dielectric multilayer film made of $SiO_2$ and $Al_2O_3$ layers is used. Therefore, the transmittance of UV light at an incident angle of 0° can be enhanced, and the light intensity of UV light in the desired wavelength range of 190 nm to 235 nm can be ensured. In addition, by reducing the total number of layers, the cost for fabricating multilayers can be reduced.

The light source for illumination 10D is provided on the ceiling 201 in the facility 200. The light source for illumination 10D is assumed to have, for example, an emission spectrum that overlaps at least a part of the wavelength range of 300 nm to 500 nm.

The controller unit 20 controls the irradiation and non-irradiation of light by the UV irradiation unit 10C. More particularly, when the inactivation apparatus 100C is an apparatus in which the target of the inactivation treatment is an object surface, the controller unit 20 controls the UV irradiation unit 10C to form a region on the surface of the object in which the irradiance of UV light having a wavelength of 200 nm to 230 nm is equal to or greater than 10 $\mu$W/cm². On the other hand, when the inactivation apparatus 100C is an apparatus in which the target of the inactivation treatment is a space, the controller unit 20 controls the UV irradiation unit 10C to form a region in which the irradiance of UV light having a wavelength of 200 nm to 230 nm is equal to or greater than 10 $\mu$W/cm² at a distance of 20 cm from the light emission surface of the UV irradiation unit 10C.

Furthermore, the controller unit 20 controls the UV irradiation unit 10C to perform intermittent lighting under a condition corresponding to the wavelength of the UV light emitted by the UV irradiation unit 10C. Here, at least a part of the period during which the intermittent lighting operation of the UV irradiation section 10C is performed is included in the period during which the light source for illumination 10D is lit (turned on).

According to the current safety standards, the maximum allowable UV exposure amount of 222 nm wavelength for one day (i.e., 8 hours) is Dmax = 22 (mJ/cm²). Therefore, the condition for intermittent lighting should be set such that the integrated light intensity (i.e., total irradiation amount) for 8 hours is within 22 (mJ/cm²).

In other words, when the irradiance on the UV-irradiated surface of the human body is W (mW/cm²) and the number of times the light emitting operation is performed in a day (i.e., 8 hours) is N, the single light emitting operation time Ta (sec) of the UV irradiation unit 10C is expressed as follows:

$$Ta \leq Dmax/(W*N) \qquad \text{formula (1)}$$

It should be noted that the value of the maximum allowable UV exposure amount Dmax in this disclosure is the current value, which may be changed in the future.

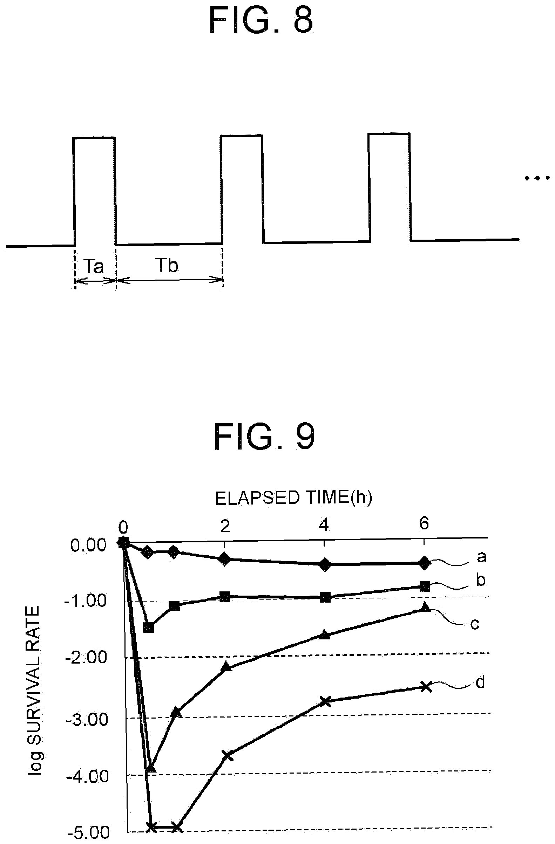

FIG. 8 is a time chart illustrating an exemplary intermittent lighting operation.

Referring to FIG. 8, Ta is the light emitting operation time for one light emitting operation, and Tb is the non-light-emitting operation time for one non-light-emitting operation. According to the present embodiment, the controller unit 20 switches the light emitting operation and the non-light-emitting operation of the UV light by controlling the power supply. Hereinafter, the light-emitting operation time Ta is referred to as the lighting time Ta and the non-light-emitting operation time Tb is referred to as the pause time Tb in the following explanation.

As shown in FIG. 8, according to the present embodiment, the inactivation apparatus may repeat the lighting operation for a predetermined time (Ta) and the pause operation for a predetermined time (Tb).

As described above, since there is a pause time during the intermittent lighting, the UV irradiation period until the irradiation amount (i.e., integrated light intensity) reaches 22 mJ/cm² is longer than that in continuous lighting, assuming that the UV irradiance is the same. For this reason, it makes it possible to enhance the possibility that UV irradiation can be carried out against the scattering of bacteria, viruses, and the like caused by the entry and exit of humans or animals into a facility or vehicle so as to increase the effectiveness of inactivation. It should be noted that once the numerical value of the maximum permissible UV exposure amount Dmax according to the safety standard is changed, the conditions for intermittent lighting shall be set based on the changed value.

In addition, intermittent lighting makes it possible to extend the usable life of the UV light source (i.e., extend the time until the UV light source needs to be replaced).

Furthermore, in the inactivation of microorganisms and viruses using UV light having a wavelength of 200 nm to 230 nm, and in particular, UV light having a wavelength of 222 nm, the same inactivation effect is obtainable regardless of the lighting operation being the continuous lighting or intermittent lighting, as long as the same amount of UV irradiation is applied. Even if the pause time of intermittent lighting is set longer, the inactivation effect does not deteriorate. This is because not only the inactivation of microorganisms and viruses, but also the proliferation of bacteria during the pause time can be effectively inhibited. Hereinafter, this point will be described in detail.

Bacterial cells contain nucleic acids (DNA and RNA) that carry genetic information. When irradiated with UV light, the nucleic acids absorb the UV light and the DNA bindings are damaged. When irradiated with the UV light, the nucleic acids absorb the light and the DNA bonds are damaged. This causes the transcriptional control of the genes to stagnate, interfering with metabolism and leading to death of bacteria. In other words, UV light does not immediately kill the bacteria themselves, but it does cause them to lose their metabolic and proliferative capabilities.

However, some bacteria cause DNA damage to be repaired when irradiated with light having a wavelength of 300 nm to 500 nm after the DNA has been damaged by UV radiation at a wavelength of 254 nm. This is due to the action of photo-reactivating enzymes (e.g., FAD (flavin adenine dinucleotide)) possessed by bacteria, and this phenomenon is called "photoreactivation of bacteria". The wavelength range of 300 nm to 500 nm also includes visible light from sunlight or white illumination, and it is known that bacterial photoreactivation progresses in a bright environment.

On the other hand, when bacteria are inactivated by UV light having a wavelength of 200 nm to 230 nm, and in particular, by UV light having a wavelength of 222 nm, the photoreactivation of bacteria does not occur even when the bacteria are irradiated with the above mentioned visible light after being irradiated with the UV light. In other words, the above described "photoreactivation of bacteria" is inhibited by UV irradiation of 222 nm wavelength.

FAD, which is a photo-reactivating enzyme, includes riboflavin, which acts on photoreactivation, and adenine nucleotide (ADP), which is further classified into adenosine and phosphate.

The absorbance of FAD is similar between UV light having a wavelength of 222 nm and UV light having a wavelength of 254 nm. On the other hand, the absorbance of riboflavin, which acts on the photoreactivation, is greater for UV light having a wavelength of 215 nm to 230 nm than for UV light having a wavelength of 254 nm. This suggests that UV light having a wavelength of 215 nm to 230 nm acts more effectively on riboflavin, thereby inhibiting its function of the photoreactivation. Furthermore, the peak absorbance value of riboflavin in the wavelength range of 200 nm to 230 nm lies near 222 nm, suggesting that UV irradiation at the wavelength of 222 nm could significantly inhibit the "photoreactivation of bacteria".

In addition, the absorbance of adenosine is greater for UV light having a wavelength of 254 nm than for UV light in the wavelength range of 218 nm to 245 nm. In other words, it can be inferred that UV light having a wavelength of 254 nm is easily absorbed by adenosine, or in other words, adenosine acts as a protective barrier against the UV light having a wavelength of 254 nm, making it difficult for riboflavin to act effectively. Therefore, UV light in the wavelength range of 218 nm to 245 nm are more likely to act effectively on riboflavin. In view of the above, UV light having a wavelength of 222 nm is a light that satisfies any of the above effective ranges and can effectively inhibit the photoreactivation effect of bacteria.

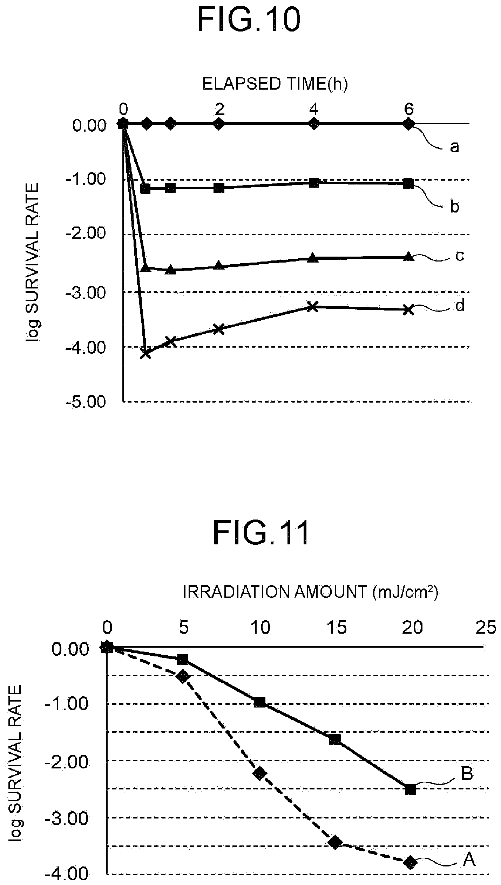

FIG. 9 is a chart exemplarily illustrates the results of the photoreactivation experiment of bacteria irradiated with UV light having a wavelength of 254 nm, and FIG. 10 is a chart exemplarily illustrates the results of the photoreactivation experiment of bacteria irradiated with UV light having a wavelength of 222 nm. Here, the bacterium to be inactivated was Staphylococcus aureus, which is easily sterilized by the UV light having a wavelength of 254 nm, and UV irradiation was performed in an environment where visible light including light having a wavelength of 300 nm to 500 nm was irradiated, and the change in the survival rate of the bacteria after UV irradiation was confirmed. The UV irradiance was set to 100 $\mu$W/cm$^2$.

Referring to FIGs. 9 and 10, the horizontal axis denotes the elapsed time (h) and the vertical axis denotes the log survival rate of the bacteria. In FIGs. 9 and 10, the experimental results a to d show the change in the survival rate of bacteria when the UV irradiation amount is 0 mJ/cm$^2$, 5 mJ/cm$^2$, 10 mJ/cm$^2$, and 15 mJ/cm$^2$, respectively.

As shown in FIG. 9, the survival rate of the bacteria increases over time. In other words, the bacteria are photo-reactivated after UV irradiation at a wavelength of 254 nm in an environment where visible light is irradiated. More particularly, the number of surviving bacteria recovers significantly in about one to two hours after visible light irradiation.

In contrast, as shown in FIG. 10, when the UV light of 222 nm wavelength is irradiated, no photoreactivation of the bacteria is observed even when visible light is irradiated. In other words, the photoreactivation of the bacteria is inhibited.

Bacteria whose photoreactivation is inhibited will not proliferate and will be inactivated, as their DNA will remain damaged. For this reason, the UV irradiation at a wavelength of 222 nm can effectively reduce the recovery and proliferation of bacteria.

As a result, an inactivation system that irradiate bacteria with UV light having a wavelength of 222 nm are particularly effective in environments where bacteria can be easily photo-reactivated, in particular, in environments where visible light including light having a wavelength of 300 nm to 500 nm is irradiated.

In the inactivation system using UV irradiation at awavelength of 254 nm, microorganisms or viruses that are not photo-reactivated (e.g., Bacillus subtilis (so-called Bacillus subtilis natto), influenza, etc.) can be effectively inactivated. On the other hand, bacteria that are photo-reactivated (e.g., Escherichia coli, Salmonella, etc.) are difficult to inactivate in an environment where visible light is irradiated. For this reason, such inactivation system is likely to create an environment in which only certain bacteria having photo-reactivating enzymes are easy to survive, and there is concern that this may increase the risk of infection by such bacteria.

For example, when Bacillus subtilis, which is harmless, coexists with Escherichia coli, which is harmful, the antibacterial substances produced by Bacillus subtilis can kill Escherichia coli. However, when Bacillus subtilis and Escherichia coli are inactivated by UV irradiation at a wavelength of 254 nm, a situation is created in which Bacillus subtilis cannot be revived but Escherichia coli can be revived. In this case, there is a concern that the risk of infection by Escherichia coli may be increased.

On the other hand, if the photoreactivation of harmful bacteria can be inhibited by irradiation with UV light having a wavelength of 200 nm to 230 nm, in particular, UV light having a wavelength of 222 nm, the risk of infection by such bacteria can be reduced.

In addition, if the photoreactivation of the bacteria can be inhibited, it makes it possible to suppress the viruses from proliferating through the bacteria.

For example, viruses that infect bacteria (i.e., bacteriophages) are known to proliferate through bacteria as a vector. Viruses may proliferate by infecting bacteria as a bacterial vector. This bacteriophage is a generic term for viruses that infect bacteria but may be harmful to humans. For example, lysogenic phages rarely have toxic or drug-resistance genes in their genomes, and it has been pointed out that these may cause harm to humans indirectly through bacteria. Examples are the toxins of cholera and diphtheria.

For this reason, inhibiting the photoreactivation of bacteria will also prevent the viruses such as phages from proliferating.

As described above, by irradiating object surfaces and spaces inside facilities or vehicles where humans or animals are present with UV light having a wavelength of 200 nm to 230 nm, and especially with UV light having a wavelength of 222 nm, it makes it possible to inactivate harmful microorganisms and viruses inside facilities or vehicles, and also to effectively suppress the photoreactivation of bacteria after UV irradiation. As a result, it makes it possible to prevent viruses such as bacteriophages from proliferating.

Furthermore, UV irradiation at a wavelength of 200 nm to 230 nm, in particular 222 nm, can inhibit the photoreactivation of bacteria, so that the inactivation effect is maintained even during a pause time (i.e., rest time) in which no UV light is irradiated. In other words, the inactivation effect of the intermittent lighting is equivalent to that of the continuous lighting.

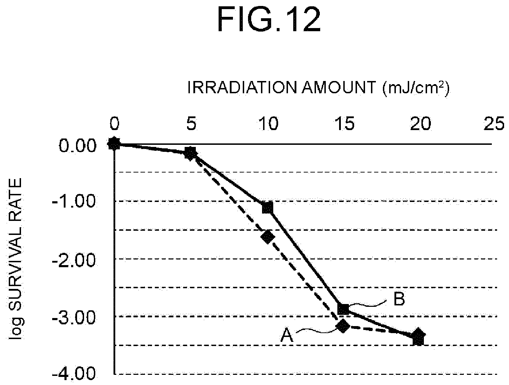

FIG. 11 is a chart exemplarily illustrating the results of comparison between the continuous lighting and the intermittent lighting using UV light having a wavelength of 254 nm, and FIG. 12 is a chart exemplarily illustrating the results of comparison between the continuous lighting and the intermittent lighting using UV light having a wavelength of 222 nm. Here, the bacterium to be inactivated was Staphylococcus aureus, and the change in the survival rate of the bacteria was confirmed between the case where the above UV light was turned on continuously and the case where the above UV light was turned on intermittently in an environment where visible light was irradiated.

Referring to FIGs. 11 and 12, the horizontal axis denotes the irradiation amount of UV light (i.e., integrated light intensity) (mJ/cm$^2$) and the vertical axis denotes the log survival rate of the bacteria. In FIGs. 11 and 12, the dashed line A denotes the results of the continuous lighting, and the solid line B denotes the results of the intermittent lighting.

The UV irradiance in the continuous lighting was set to 100 ($\mu$W/cm$^2$).

The conditions for the intermittent lighting were set to lighting time Ta = 50 (sec), pause time Tb = 59 minutes and 10 seconds (i.e., 3,550 (sec)), and lighting duty ratio = 1.39 (%). The lighting duty ratio is the ratio of the lighting time Ta to the total sum of the lighting time Ta and the pause time Tb, and is the value expressed by Td = Ta/(Ta + Tnb). The UV irradiance during lighting was set to 100 ($\mu$W/cm$^2$), and the irradiation amount of UV light per lighting operation was set to 5 (mJ/cm$^2$).

As shown in FIG. 11, in the case of UV irradiation at a wavelength of 254 nm, the inactivation effect of the intermittent lighting is inferior to that of the continuous lighting. It is considered to be due to that fact that the bacteria are photo-reactivated during the pause time of the intermittent lighting. Thus, UV irradiation at a wavelength of 254 nm with the intermittent lighting cannot ensure the inactivation of the bacteria because the bacteria exert a photoreactivation effect in case of the UV irradiation at a wavelength of 254 nm.

On the other hand, as shown in FIG. 12, in the case of UV irradiation at a wavelength of 222 nm, the inactivation effect is equivalent between the intermittent lighting and the continuous lighting, because the photoreactivation of bacteria is inhibited.

Furthermore, as shown in FIGs. 11 and 12, when the bacterium to be inactivated is Staphylococcus aureus, the inactivation effect of using UV light at a wavelength of 254 nm is higher than that of using UV light at a wavelength of 222 nm at any UV irradiation amount in the continuous lighting. In contrast, in the case of the intermittent lighting, the inactivation effect is higher when 222 nm wavelength UV light is used at any UV irradiation amount (irradiance level).

In the intermittent lighting using UV light at a wavelength of 222 nm, the inactivation effect does not deteriorate even with a longer pause time.

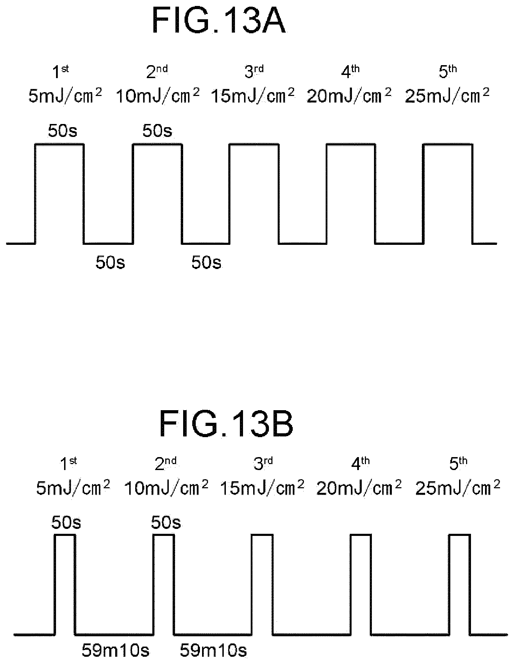

FIG. 13A is a time chart exemplarily illustrating an Operation Example 1 of the intermittent lighting using UV light having a wavelength of 222 nm, and FIG. 13B is a time chart exemplarily illustrating an Operation Example 2 of the intermittent lighting using UV light having a wavelength of 222 nm. The UV irradiance at the time of lighting and the lighting time Ta per lighting (i.e., at one time) are the same between the Operation Example 1 and the Operation Example 2, and only the pause time Tb per pause (i.e., at one time) is different.

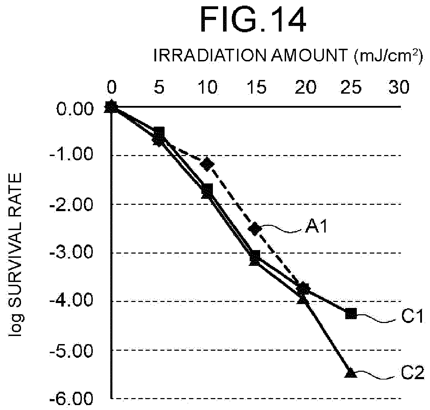

FIG. 14 is a chart exemplarily illustrating the inactivation effect when the intermittent lighting is performed in the Operating Examples 1 and 2. Referring to FIG. 14, the experimental results C1 and C2 denote the change in the survival rate of bacteria when the intermittent lighting is performed in the Operation Examples 1 and 2, respectively. The experimental result A1 denotes the change in the survival rate of bacteria when the continuous lighting is performed with the same UV irradiance during lighting as in the Operation Examples 1 and 2.

Here, as shown in FIG. 13A, the Operation Example 1 has a lighting time Ta = 50 (sec) and a pause time Tb = 50 (sec), that is, the lighting duty ratio is set to 50%. As shown in FIG. 13B, the Operation Example 2 has the lighting time Ta = 50 (sec) and the pause time Tb = 59 minutes and 10 seconds (i.e., 3,550 (sec)), that is, the lighting duty ratio is set to 1.39%.

In both Operation Examples 1 and 2, the UV irradiance at the time of lighting is setto 100 ($\mu$W/cm$^2$). In other words, the UV irradiation amount by the first lighting operation (i.e., per one lighting operation) is 5 mJ/cm$^2$ in both cases, and thereafter, the UV irradiation amount through the second, third, ... lighting operations is set to 10 mJ/cm$^2$, 15 mJ/cm$^2$, ..., respectively.

Thus, although the UV irradiation amount by one lighting operation is the same between the intermittent lighting of the Operation Example 1 and that of the Operation Example 2, the pause time of the Operation Example 2 is longer than that of the Operation Example 1. Nevertheless, as shown in FIG. 14, the inactivation effects of the Operation Examples 1 and 2 are substantially the same, and it can be confirmed that the inactivation effect does not deteriorate even if the pause time is set to be longer.

It can also be confirmed that the inactivation effect of the Operation Examples 1 and 2 is substantially the same as the inactivation effect of the continuous lighting at the same UV irradiance.

Also examined was the inactivation effect of low UV irradiance ($\mu$W/cm$^2$) at the time of lighting.

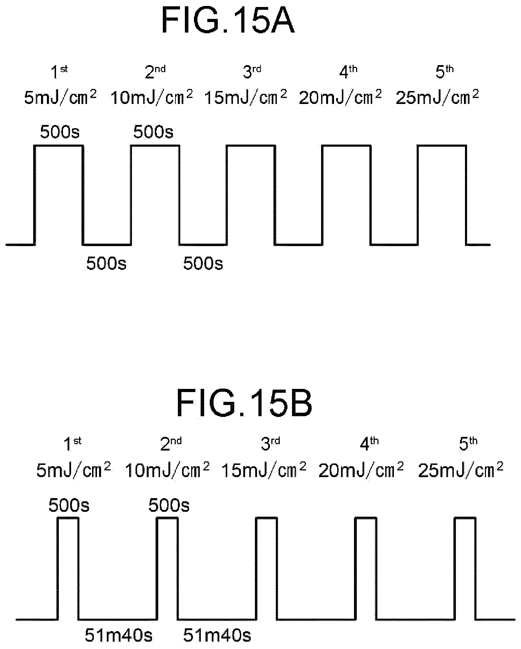

FIG. 15A is a time chart exemplarily illustrating an Operation Example 3 of the intermittent lighting using UV light having a wavelength of 222 nm, and FIG. 15B is a time chart exemplarily illustrating an Operation Example 4 of the intermittent lighting using UV light having a wavelength of 222 nm. The UV irradiance at the time of lighting and the lighting time Ta at one time are the same between the Operation Example 3 and the Operation Example 4, and only the pause time Tb at one time is different therebetween.

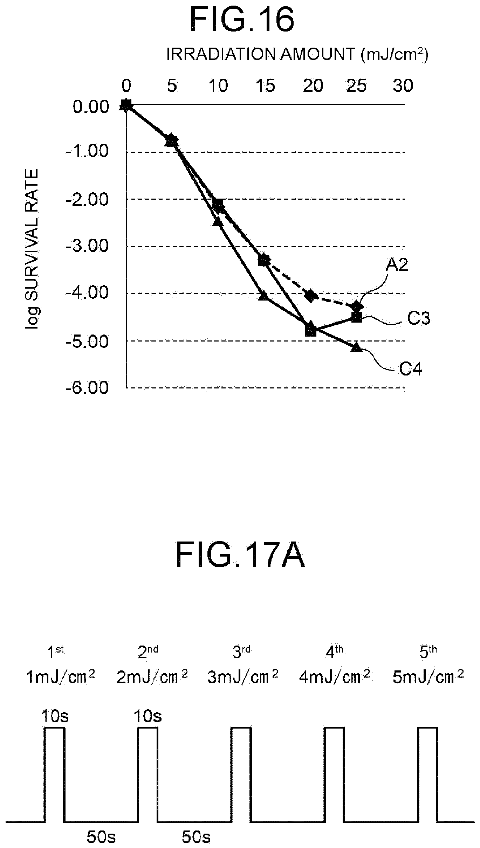

FIG. 16 is a chart exemplarily illustrating the inactivation effect when the intermittent lighting is performed in the Operating Examples 3 and 4. Referring to FIG. 16, experimental results C3 and C4 denote the change in the survival rate of bacteria when the intermittent lighting is performed in the Operating Examples 3 and 4, respectively. The experimental result A2 denotes the change in the survival rate of bacteria when the continuous lighting is performed with the same UV irradiance at the time of lighting as in the Operation Examples 3 and 4.

Here, as shown in FIG. 15A, the Operation Example 3 has the lighting time Ta = 500 (sec) and the pause time Tb = 500 (sec), that is, the lighting duty ratio is set to 50%.

As shown in FIG. 15B, the Operation Example 4 has the lighting time Ta = 500 (sec) and the pause time Tb = 51 minutes and 40 seconds (i.e., 3,100 (sec)), that is, the lighting duty ratio is set to 13.9%.

In both of the Operation Examples 3 and 4, the UV irradiance at the time of lighting is set to 10 ($\mu$W/cm$^2$). In other words, the UV irradiation amount by the first lighting operation (i.e., per one lighting operation) is 5 mJ/cm$^2$ in each case, which is the same as in the Operation Examples 1 and 2 above, and thereafter, the UV irradiation amount through the second, third, ... lighting operations is set to 10 mJ/cm$^2$, 15 mJ/cm$^2$, ..., respectively.

Thus, although the UV irradiation amount by one lighting operation is the same between the intermittent lighting of the Operation Examples 3 and 4 and that of the Operation Examples 1 and 2, the UV irradiance in the Operation Examples 3 and 4 is lower than that of the Operation Examples 1 and 2. Nevertheless, as shown in FIGs. 14 and 16, similarly to the inactivation effect of the Operation Examples 1 and 2, it can be confirmed that the inactivation effects of the Operation Examples 3 and 4 with relatively low UV irradiance at the time of lighting does not deteriorate even if the pause time is set to be longer, and the inactivation effect can be maintained.

It can also be confirmed that the inactivation effect of the Operation Examples 3 and 4 is substantially the same as the inactivation effect of the continuous lighting at the same UV irradiance.

As described above, the inactivation apparatus 100C according to the present embodiment includes the ultraviolet light irradiation unit (i.e., UV irradiation unit) 10C that irradiates object surfaces and spaces in the facility 200 where humans or animals are present with UV light having a wavelength of 200 nm to 230 nm that inactivates microorganisms and/or viruses harmful to the human body or animals. The inactivation apparatus 100C also includes the controller unit 20 that controls the irradiation and non-irradiation of UV light by the UV irradiation unit 10C. The controller unit 20 controls the UV irradiation unit 10C to perform the intermittent lighting such that the light emitting operation (lighting operation) and the non-light emitting operation (pause operation) by the UV irradiation unit 10C are alternately repeated in accordance with the wavelength of the UV light irradiated from the UV irradiation unit 10C.

More particularly, the controller unit 20 controls the intermittent lighting by the UV light having a wavelength of 200 nm to 230 nm in the facility where humans or animals are present, under the condition that the daily UV irradiation amount (i.e., integrated light intensity) is within the maximum allowable UV exposure amount Dmax specified by the ACGIH. It makes it possible to inactivate harmful microorganisms and viruses present in the facility while appropriately suppressing the adverse effects of UV light on humans and animals.

In addition, since the intermittent lighting is performed, the period until the integrated light intensity of UV light reaches the maximum allowable UV exposure amount Dmax can be longer than the period until the integrated light intensity of UV light reaches the maximum allowable UV exposure amount Dmax with continuous lighting at the same UV irradiance. As a result, it makes it possible to enhance the possibility of inactivating harmful microorganisms and viruses scattered in the facility, and to lengthen the usable life of the UV irradiation unit 10C (i.e., the time until the UV light source needs to be replaced) as compared to the case of continuous lighting.

In addition, the UV irradiation unit 10C can effectively inhibit the photoreactivation of bacteria by irradiating the target object or space with UV light having a wavelength of 222 nm. Therefore, even in an environment where visible light is irradiated from the light source for illumination 10D, it makes it possible to prevent the bacteria, which have been inactivated during the lighting time, from being photo-reactivated during the pause time without the UV irradiation so as to maintain the inactivation effect. In other words, the inactivation effect of the intermittent lighting is equivalent to the inactivation effect of the continuous lighting.

Here, the conditions for intermittent lighting can be set according to the integrated light intensity by one lighting operation, the irradiance during lighting operation, the lighting time Ta, the pause time Tb, and the lighting duty ratio Td.

For example, the integrated light intensity per one lighting operation may be equal to or less than 10 $mJ/cm^2$. Conventionally, in an inactivation system, the integrated light intensity of UV light has been set to be more than the equivalent of the amount of energy required for sterilization in order to significantly reduce the bacteria to be sterilized (for example, 99.9% sterilization) with a single UV irradiation. In addition, taking the problem of the photoreactivation of bacteria into consideration, the need to further increase the integrated light intensity per one time has been conventionally considered when intermittent lighting is used.

On the other hand, according to the present embodiment, by intermittent lighting using light in the wavelength range that can inhibit the photoreactivation of bacteria, a higher inactivation effect can be attained even when the integrated light intensity per one time is kept low. More particularly, even when the amount of UV light irradiated per one time is lower than the amount of light that can inactivate the microorganism or virus to be inactivated, the microorganism or virus to be inactivated can be appropriately inactivated by repeating the intermittent lighting. For example, although the irradiation amount of UV light required for 99.9% sterilization of Staphylococcus aureus is about 15 $mJ/cm^2$, even if the intermittent lighting is performed with an integrated light intensity of 5 $mJ/cm^2$ per one lighting operation, the inactivation effect can be appropriately attained, as shown in FIGs. 14 and 16.

Furthermore, the integrated light intensity per one lighting operation may be 5 $mJ/cm^2$ or less.

FIG. 17A is a time chart exemplarily illustrating an Operation Example 5, in which the integrated light intensity by one lighting operation is set to 1 $mJ/cm^2$. In the Operation Example 5, the lighting time Ta = 10 (sec), the pause time Tb = 50 (sec), and the UV irradiance during lighting is set to 100 ($\mu W/cm^2$). In other words, the integrated light intensity by the first lighting operation is 1 $mJ/cm^2$, and thereafter, the integrated light intensity through the second, third, ... lighting operations is set to 2 $mJ/cm^2$, 3 $mJ/cm^2$, ..., respectively. The bacterium to be inactivated was Staphylococcus aureus.

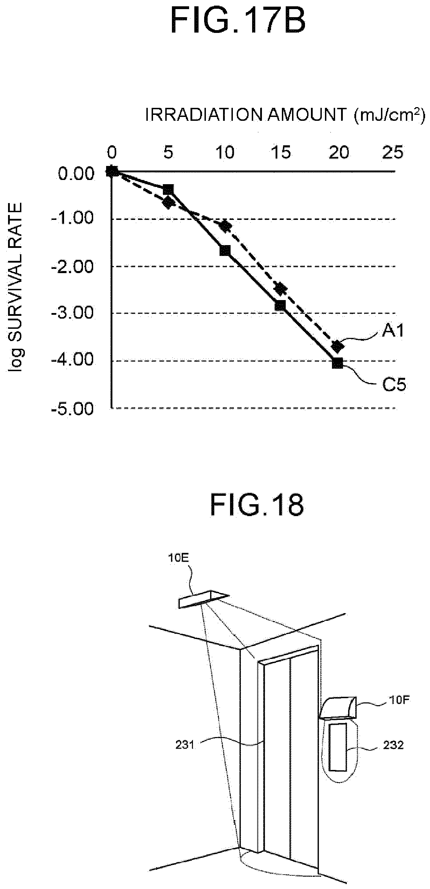

FIG. 17B is a chart exemplarily illustrating the inactivation effect when the intermittent lighting is performed in the Operation Example 5. In FIG. 17B, the experimental result C5 denotes the change in the survival rate of bacteria when the intermittent lighting is performed in the Operation Example 5, and the experimental result A1 denotes the change in the survival rate of bacteria when the continuous lighting is performed with the same UV irradiance at the time of lighting as in the Operation Example 5. This experimental result A1 is the same as the experimental result A1 shown in FIG. 14. As shown in FIG. 17B, the inactivation effect can be appropriately attained even when the integrated light intensity by one lighting operation is 1 $mJ/cm^2$, which is less than 5 $mJ/cm^2$.

Also, the integrated light intensity per one lighting operation may be set to a lower value. For example, the integrated light intensity of a single lighting operation may be set to 1 $mJ/cm^2$.

The lighting duty ratio Td may be set to, for example, 50% or less. Also in this case, the inactivation effect can be appropriately attained as shown in FIGs. 14 and 16. In addition, by setting the lighting duty ratio Td to 50% or less, it makes it possible to extend the time for which the inactivated environment can be maintained more than twice with the same integrated light intensity as compared to the case of the continuous lighting.

The lighting duty ratio Td may be set to 25% or less or 10% or less to maintain a more inactivated environment.

Furthermore, the lighting duty ratio Td may be set to be between 1% and 5%, for example. In this case as well, the inactivation effect can be appropriately attained as shown in the experimental results C2 in FIG. 14 and C4 in FIG. 16. In addition, in this case, it makes it possible to further extend the time for which the inactivated environment can be maintained.

Yet furthermore, the lighting time Ta per one time may be equal to or less than one minute. In this case as well, the inactivation effect can be appropriately attained as shown in FIG. 14. According to the present embodiment, since an excimer lamp (e.g., KrCl excimer lamp, KrBr excimer lamp, and the like), which has a shorter rise time of light output than the conventional mercury lamp, is used as the UV light source, even when the lighting time Ta is one minute or less, it makes it possible to attain the stable light output and effectively create the inactivation environment.

According to the present embodiment, it has been described the case where the excimer lamp is used as the UV light source with shorter rise time of light output, but solid-state light sources (light-emitting diodes (LEDs), laser diodes (LDs), and the like) may also be used.

In the operation cycle of the lighting operation and the pause operation, the pause time Tb of two hours or more may be set for at least a part of the operation cycle. Since the present embodiment can inhibit the photoreactivation of bacteria, the inactivation effect can be appropriately sustained, even when the pause time Tb is set longer than the time required for the photoreactivation of bacteria. It should be noted that, as shown in FIG. 9, when irradiating with UV light having a wavelength of 254 nm, the time required for photoreactivation of bacteria is about 1 to 2 hours.

On the other hand, in the operation cycle of the lighting operation and the pause operation, it is preferable that the pause time Tb of one time for the UV irradiation unit is set to one hour or less. In order to prevent the spread of infection in a facility or a vehicle, it is preferable to shorten the pause time Tb during a period when humans or animals are coming and going.

For example, the airborne infection of viruses is supposed to spread in a state that viruses are attached to minute aerosols of less than 1 $\mu$m in the air. In this case, the time that the minute aerosol floats in the air is long, and depending on the type of virus, some viruses have a survival time in the aerosol of more than one hour (for example, new coronaviruses).

In order to effectively irradiate such viruses with UV light, it is preferable to control the pause time Tb to be one hour or less. It makes it possible to appropriately inactivate viruses surviving in the aerosol so as to reduce the risk of infection when a human newly enters the facility or the vehicle. In addition, by setting the pause time Tb further shorter, it makes it possible to perform the UV irradiation multiple times in the aerosol so as to increase the inactivation effect.

In addition, the route of infection through humans and animals needs to be taken into consideration in order to suppress the spread of infection.

When humans sneeze, the droplets can be roughly classified into large particles of 5 $\mu$m or more and small particles of less than 5 $\mu$m (i.e., droplet nuclei). Here, the smaller particles of less than 5 $\mu$m have a slower falling speed, which is about 0.06 cm/s to 1.5cm/s. Assuming a fall speed of 0.06 cm/s, it would take about 27 minutes for a particle less than 5 $\mu$m to fall by one meter.

Therefore, the pause time Tb may be set to 25 minutes or less, for example. In this case, it makes it possible to appropriately irradiate small particles (i.e., droplet nuclei) of less than 5 $\mu$m, which tend to drift in the air, with UV light before they fall on the floor so as to effectively inactivate airborne viruses and bacteria. After the droplet nuclei have fallen to the ground, the accumulation of sediment (dust, etc.) around the viruses and bacteria may act as a barrier to the UV light. In contrast, by irradiating with UV light in the air with little amount of shielding, the bacteria and viruses can be expected to be reduced.

When the viruses are irradiated with UV light multiple times in the air, a pause time Tb of 10 minutes or less is preferable.

According to the present embodiment, the controller unit 20 may be configured to change the operation cycle of the lighting operation and the pause operation by the UV irradiation unit 10C in accordance with the proliferation situation of microorganisms and/or viruses harmful to humans or animals in the facility or vehicle. In this case, the conditions of the intermittent lighting (including the integrated light intensity per one lighting operation, the irradiance at the time of the lighting operation, the lighting time Ta, the pause time Tb, and the lighting duty ratio Td) may be individually and freely changeable, or the controller unit 20 may be configured to switch between a plurality of different pre-set operational modes.

For example, during periods when there is little or no human traffic, such as facility holiday periods or off-season periods, there is little need for frequent UV irradiation. Therefore, in such periods, the pause time Tb may be set to be longer.

On the other hand, for example, it can be determined to be a situation in which microorganisms and/or viruses harmful to the human body or animals tend to proliferate, if it is observed to be in a time zone of day in which there is a lot of human or animal traffic, a situation in which the proliferating environment of bacteria is easily established, or a situation in which an infectious disease is spreading. In such a situation, the operation cycle may be changed automatically or manually, for example, such that the pause time Tb becomes shorter. In the case of changing the operation cycle automatically, for example, the time of day, temperature, humidity, frequency of human or animal traffic, and other conditions may be detected by sensors, and the operation cycle may be changed by judging the proliferating situation based on the detected signals. In addition, the controller unit 20 may controls the intermittent lighting to accumulate data on changes over time in the environment where UV light is irradiated (including temperature, humidity, frequency of human or animal traffic, and the like) and to change the operation cycle based on the accumulated data, and artificial intelligence may be used in combination at that time. On the other hand, in the case of manually changing the operation cycle, a signal indicating the operation mode selected by the user according to the proliferation status may be received, and the operation cycle may be changed based on the received signal. The signal here may be a wireless signal using a remote control or the like, or it may be a wired signal from a wired switch operation.

Hereinafter, use cases of the inactivation apparatus will be exemplified in detail.

FIG. 18 is a schematic view illustrating an example where ultraviolet light irradiation units (i.e., UV irradiation units) 10E and 10F are installed at a doorway 231, or at an operating unit (e.g., operating panel) 232 provided in the vicinity of the doorway 231. In this case, the UV irradiation unit 10E can irradiate UV light toward the traffic space of the doorway 231. In addition, the UV irradiation unit 10F can irradiate UV light toward the operating unit 232 provided in the vicinity of the doorway 231. Since the operating panel 232 is likely to be touched by humans, the UV irradiation unit 10F may irradiate UV light toward the space including the operating unit 232.

It makes it possible to irradiate UV light onto bacteria and viruses floating in the traffic space of the doorway 231, onto bacteria and viruses attached to humans coming and going through the doorway 231 and also toward the operating unit 232 so as to inactivate all of them.

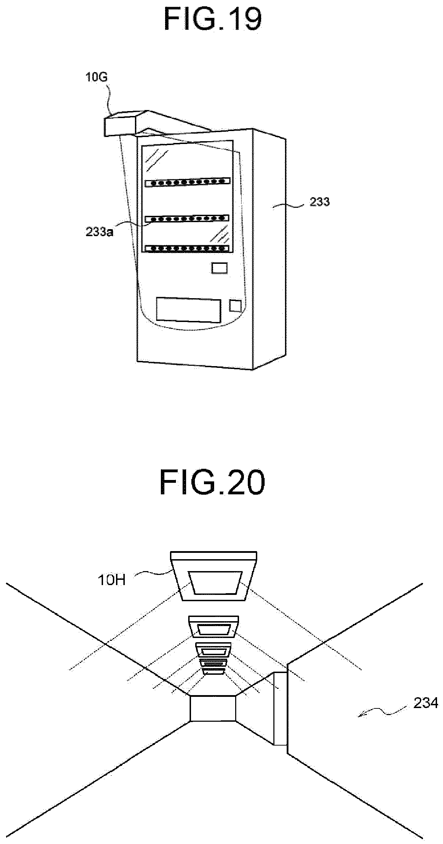

FIG. 19 is a schematic view illustrating an example where an ultraviolet light irradiation unit (i.e., UV irradiation unit) 10G is installed in an equipment operated by a human (in this case, a vending machine 233). In this case, the UV irradiation unit 10G irradiates UV light toward the operating buttons 233a on the equipment and the space including the operating buttons 233a.

It makes it possible to irradiate UV light onto bacteria and viruses attached to the operating buttons 233a, or onto fingers of a human before touching the operating buttons 233a. Therefore, it is expected to reduce the possibility of bacteria and viruses attaching to the operating button 233a. In addition, even when bacteria or viruses attach to the operating button 233a, it makes it possible to inactivate the bacteria or viruses.

The same configuration can be applied to any equipment that is operated by humans, such as a ticket vending machine and an ATM, which is equipped with an operating unit (e.g., an operation button, switch, touch panel, or the like). By irradiating UV light onto bacteria and viruses attaching to the surface of the operating unit or onto the fingers of humans before they touch the operating unit, it is expected to prevent the risk of spreading infections caused by bacteria and viruses through the operating unit in advance.

FIG. 20 is a schematic view illustrating an example of an ultraviolet light irradiation unit (i.e., UV irradiation unit) 10H installed at the upper part (e.g., ceiling) of the passage 234. In this case, the UV irradiation unit 10H irradiates the target space above the passage 234 with UV light. This is in particular expected to inactivate bacteria and viruses floating above the passage 234.

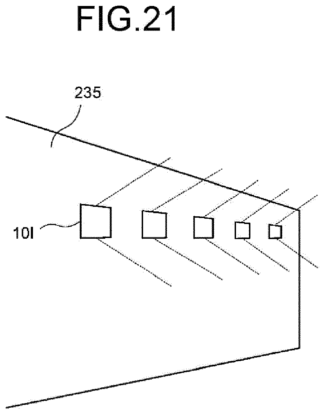

FIG. 21 is a schematic view illustrating an example of an ultraviolet light irradiation unit (i.e., UV irradiation unit) 10I on a wall 235. In this case, the UV irradiation unit 10I irradiates UV light toward the space facing the wall 235. This is in particular expected to inactivate bacteria and viruses floating in the vicinity of the wall 235.

Although specific embodiments have been described above, the embodiments described are illustrative only and are not intended to limit the scope of the present invention. The apparatus and method described herein may be embodied in other forms than as described above. In addition, it is also possible to appropriately omit, substitute, or modify the above described embodiments without departing from the scope of the present invention. Embodiments with such omissions, substitutions and modifications fall within the scope of the appended claims and equivalents thereof and also fall within the technical scope of the present invention.

REFERENCE SIGNS LIST

10A to 10C: UV Irradiation Unit; 10D: Light Source for Illumination; 11: Light Emission Surface; 20: Controller Unit; 100A to 100C: Inactivation Apparatus; 200: Facility; 201: Ceiling; 210: Floor; 220: Target Object; 230: Target Space; 300: Human

**Claims**

1. An inactivation apparatus for inactivating microorganisms and/or viruses on a surface of an object, comprising:

    an ultraviolet light irradiation unit having a light emission surface configured to emit light including ultraviolet light having a wavelength that inactivates the microorganisms and/or viruses; and
    a controller unit configured to control irradiation of the light by the ultraviolet light irradiation unit, the ultraviolet light included in the light emitted from the light emission surface being ultraviolet light having a center wavelength of 200 nm to 230 nm, and
    the controller unit controlling the ultraviolet light irradiation unit to irradiate the surface of the object with the ultraviolet light to form a region, on the surface of the object, in which irradiance of the ultraviolet light having the wavelength of 200 nm to 230 nm becomes equal to or greater than 10 $\mu$W/cm$^2$.

2. An inactivation apparatus for inactivating microorganisms and/or viruses floating in a target space, comprising:

    an ultraviolet light irradiation unit having a light emission surface configured to emit light including ultraviolet light having a wavelength that inactivates the microorganisms and/or viruses; and
    a controller unit configured to control irradiation of the light by the ultraviolet light irradiation unit, the ultraviolet light included in the light emitted from the light emission surface being ultraviolet light having a center wavelength of 200 nm to 230 nm, and

the controller unit controlling the ultraviolet light irradiation unit to irradiate the target space with the ultraviolet light to form a region in which irradiance of the ultraviolet light having the wavelength of 200 nm to 230 nm becomes equal to or greater than 10 $\mu$W/cm$^2$ at a distance of 20 cm from the light emission surface.

3. The inactivation apparatus according to Claim 1 or 2, wherein
the ultraviolet light irradiation unit emits the ultraviolet light having a center wavelength of 222 nm.

4. The inactivation apparatus according to any one of Claims 1 to 3, wherein
the controller unit controls lighting by the ultraviolet light irradiation unit such that an irradiance value of the ultraviolet light by the ultraviolet light irradiation unit varies over time or periodically between a first irradiance value and a second irradiance value that is lower than the first irradiance value.

5. The inactivation apparatus according to any one of Claims 1 to 3, wherein
the controller unit controls the ultraviolet light irradiation unit to alternately repeat an emitting operation and a non-emitting operation of the light by the ultraviolet light irradiation unit and to intermittently perform light emission from the light emission surface.

6. An inactivation method of inactivating microorganisms and/or viruses on a surface of an object, comprising the steps of:

controlling an ultraviolet light irradiation unit to emit light having a center wavelength of 200 nm to 230 nm, as ultraviolet light having a wavelength that inactivates the microorganisms and/or viruses, from a light emission surface of the ultraviolet light irradiation unit; and
irradiating the surface of the object with the ultraviolet light to form a region, on the surface of the object, in which irradiance of the ultraviolet light having the wavelength of 200 nm to 230 nm becomes equal to or greater than 10 $\mu$W/cm$^2$.

7. An inactivation method of inactivating microorganisms and/or viruses floating in a target space, comprising the steps of:

controlling an ultraviolet light irradiation unit to emit light having a center wavelength of 200 nm to 230 nm, as ultraviolet light having a wavelength that inactivates the microorganisms and/or viruses, from a light emission surface of the ultraviolet light irradiation unit; and
irradiating the target space with the ultraviolet light to form a region in which irradiance of the ultraviolet light having the wavelength of 200 nm to 230 nm becomes equal to or greater than 10 $\mu$W/cm$^2$ at a distance of 20 cm from the light emission surface.

8. The inactivation method according to Claim 6 or 7, wherein
lighting by the ultraviolet light irradiation unit is controlled such that an irradiance value of the ultraviolet light by the ultraviolet light irradiation unit varies over time or periodically between a first irradiance value and a second irradiance value that is lower than the first irradiance value.

9. The inactivation method according to any one of Claims 6 to 8, wherein
the ultraviolet light irradiation unit is controlled to alternately repeat an emitting operation and a non-emitting operation of the light by the ultraviolet light irradiation unit and to intermittently perform light emission from the light emission surface.

# FIG. 1

# FIG. 2

# FIG. 3

IRRADIANCE (µW/cm²)

# FIG. 4

IRRADIANCE (µW/cm²)

# FIG. 5

# FIG. 6

# FIG. 7

# FIG. 8

# FIG. 9

# FIG.10

# FIG.11

# FIG.12

# FIG.13A

1st      2nd      3rd      4th      5th

$5mJ/cm^2$   $10mJ/cm^2$   $15mJ/cm^2$   $20mJ/cm^2$   $25mJ/cm^2$

50s        50s

50s        50s

# FIG.13B

1st      2nd      3rd      4th      5th

$5mJ/cm^2$   $10mJ/cm^2$   $15mJ/cm^2$   $20mJ/cm^2$   $25mJ/cm^2$

50s        50s

59m10s      59m10s

# FIG.14

IRRADIATION AMOUNT (mJ/cm²)

log SURVIVAL RATE

A1

C1

C2

# FIG.15A

| 1st | 2nd | 3rd | 4th | 5th |
| 5mJ/cm² | 10mJ/cm² | 15mJ/cm² | 20mJ/cm² | 25mJ/cm² |

500s    500s

500s    500s

# FIG.15B

| 1st | 2nd | 3rd | 4th | 5th |
| 5mJ/cm² | 10mJ/cm² | 15mJ/cm² | 20mJ/cm² | 25mJ/cm² |

500s    500s

51m40s    51m40s

# FIG.16

# FIG.17A

# FIG.17B

# FIG.18

# FIG.19

# FIG.20

# FIG.21

Europäisches
Patentamt
European
Patent Office
Office européen
des brevets

# EUROPEAN SEARCH REPORT

**Application Number**

EP 21 18 5996

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 421 053 A1 (BOEING CO [US]) 2 January 2019 (2019-01-02) * paragraph: [0001], [0011], [0028-0030], [0032] * ----- | 1,3-5 | INV. A61L2/00 A61L9/20 A61L2/10 |
| X | WO 00/28552 A1 (CLEAN EARTH TECH LLC [US]; CURRY RANDY DALE [US]; GOLDEN JEFFRY [US]) 18 May 2000 (2000-05-18) * page 16: line 27-31; page 17: line 1-26; claim: 21, 25, 27 * ----- | 1,2,4-9 | |
| X | US 2010/007492 A1 (RESSLER BARRY [US] ET AL) 14 January 2010 (2010-01-14) * paragraph: [0041], [0048] * ----- | 1-9 | |
| X | US 2018/236115 A1 (YERBY EARL [US]) 23 August 2018 (2018-08-23) * paragraph: [0035], [0036], [0041] * ----- | 1,2,4-9 | |

| | |
|---|---|
| | **TECHNICAL FIELDS SEARCHED (IPC)** |
| | A61L |

The present search report has been drawn up for all claims

| Place of search | Date of completion of the search | Examiner |
|---|---|---|
| Munich | 9 December 2021 | Nania, Manuela |

EPO FORM 1503 03.82 (P04C01)

## ANNEX TO THE EUROPEAN SEARCH REPORT
## ON EUROPEAN PATENT APPLICATION NO.

EP 21 18 5996

This annex lists the patent family members relating to the patent documents cited in the above-mentioned European search report.
The members are as contained in the European Patent Office EDP file on
The European Patent Office is in no way liable for these particulars which are merely given for the purpose of information.

09-12-2021

| Patent document cited in search report | | Publication date | Patent family member(s) | | Publication date |
|---|---|---|---|---|---|
| EP 3421053 | A1 | 02-01-2019 | BR 102018013043 | A2 | 15-01-2019 |
| | | | CA 3006530 | A1 | 26-12-2018 |
| | | | CN 109121273 | A | 01-01-2019 |
| | | | EP 3421053 | A1 | 02-01-2019 |
| | | | JP 2019030635 | A | 28-02-2019 |
| | | | US 2018373157 | A1 | 27-12-2018 |
| | | | US 2019171111 | A1 | 06-06-2019 |
| | | | US 2021063891 | A1 | 04-03-2021 |
| WO 0028552 | A1 | 18-05-2000 | AU 1473400 | A | 29-05-2000 |
| | | | EP 1138047 | A2 | 04-10-2001 |
| | | | IL 165884 | A | 03-06-2007 |
| | | | IL 165885 | A | 03-06-2007 |
| | | | IL 165886 | A | 03-06-2007 |
| | | | WO 0028552 | A1 | 18-05-2000 |
| US 2010007492 | A1 | 14-01-2010 | EP 2303338 | A1 | 06-04-2011 |
| | | | US 2010007492 | A1 | 14-01-2010 |
| | | | WO 2009149020 | A1 | 10-12-2009 |
| US 2018236115 | A1 | 23-08-2018 | AU 2013239823 | A1 | 16-10-2014 |
| | | | AU 2016200157 | A1 | 04-02-2016 |
| | | | CA 2868795 | A1 | 03-10-2013 |
| | | | EP 2830670 | A1 | 04-02-2015 |
| | | | US 2013256560 | A1 | 03-10-2013 |
| | | | US 2016206766 | A1 | 21-07-2016 |
| | | | US 2018236115 | A1 | 23-08-2018 |
| | | | WO 2013148755 | A1 | 03-10-2013 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020141843 A
- JP 2018130131 A

- JP 2018517488A W